# EUROPEAN PATENT SPECIFICATION

(11) **EP 2 484 379 B1**
(45) Date of publication and mention of the grant of the patent: **13.07.2016**
(21) Application number: 10818899.6
(22) Date of filing: 27.09.2010
(51) Int. Cl.: A61K 39/395, A61P 9/10, C07K 16/18, C12P 21/08, C07K 16/24

(54) **AGENT FOR PREVENTION OF ATHEROSCLEROSIS**
MITTEL ZUR VERHINDERUNG VON ARTERIOSKLEROSE
AGENT POUR LA PRÉVENTION DE L'ATHÉROSCLÉROSE

(30) Priority: 28.09.2009 JP 2009223472
(43) Date of publication of application: 08.08.2012
(73) Proprietor: National University Corporation Okayama University, Kita-ku Okayama-shi Okayama 700-8530 (JP); Sowakai Medical Foundation, Kurashiki-shi, Okayama 710-0051 (JP)
(72) Inventor: NISHIBORI, Masahiro, Okayama-shi Okayama 700-8558 (JP); TAKAHASHI, Hideo, Okayama-shi Okayama 700-8558 (JP); MORI, Shuji, Okayama-shi Okayama 700-8558 (JP); LIU, Keyue, Okayama-shi Okayama 700-8558 (JP); TOMONO, Yasuko, Okayama-shi Okayama 701-0202 (JP)
(74) Representative: Müller-Boré & Partner Patentanwälte PartG mbB
(86) International application number: PCT/JP2010/066683
(87) International publication number: WO 2011/037227

(56) References cited:
- WO-A1-2007/135992
- JP-A- 2004 523 579
- JP-A- 2009 096 739
- TREUTIGER C J ET AL: "HIGH MOBILITY GROUP 1 B-BOX MEDIATES ACTIVATION OF HUMAN ENDOTHELIUM", JOURNAL OF INTERNAL MEDICINE, BLACKWELL PUBLISHING LTD, GB, vol. 254, no. 4, 1 October 2003 (2003-10-01), pages 375-385, XP009076827, ISSN: 0954-6820, DOI: 10.1046/J.1365-2796.2003.01204.X
- YAN X X ET AL: "Increased serum HMGB1 level is associated with coronary artery disease in nondiabetic and type 2 diabetic patients", ATHEROSCLEROSIS, ELSEVIER IRELAND LTD, IE, vol. 205, no. 2, 14 December 2008 (2008-12-14), pages 544-548, XP026377316, ISSN: 0021-9150 [retrieved on 2008-12-14]
- LI W ET AL: "Role of HMGB1 in cardiovascular diseases", CURRENT OPINION IN PHARMACOLOGY, ELSEVIER SCIENCE PUBLISHERS, NL, vol. 6, no. 2, 17 February 2006 (2006-02-17), pages 130-135, XP028058515, ISSN: 1471-4892, DOI: 10.1016/J.COPH.2005.10.010 [retrieved on 2006-04-01]
- BOBIK ALEX ET AL: "A Proatherogenic Role for HMGB1 in Atherosclerosis", CIRCULATION; 82ND SCIENTIFIC SESSION OF THE AMERICAN-HEART-ASSOCIATION, LIPPINCOTT WILLIAMS & WILKINS, US; ORLANDO, FL, USA, vol. 120, no. 18, Suppl. 2, 3 November 2009 (2009-11-03), page S1155, XP009168427, ISSN: 0009-7322
- LIU, K. ET AL.: 'Anti-high mobility group box 1 monoclonal antibody ameliorates brain infarction induced by transient ischemia in rats' FASEB J vol. 21, no. 14, 2007, pages 3904 - 3916, XP002556545
- WAUTIER, J.L. ET AL.: 'Blood cells and vascular cell interactions in diabetes' CLIN HEMORHEOL MICROCIRC vol. 25, no. 2, 2001, pages 49 - 53, XP008158296

## Description

### TECHNICAL FIELD

The invention relates to an agent for suppressing atherosclerosis, a method for suppressing atherosclerosis and an anti-HMGB1 monoclonal antibody, used for suppressing atherosclerosis.

### BACKGROUND ART

Atherosclerosis is a disease in which deposition called as atherosclerosis plaque develops in the intimal layer of arterial blood vessel to decrease or block blood flow. Further, atherosclerosis plaque may become hard and rupture due to digestion of fibrous cap, accumulation of calcium ion, or the like. In the ruptured atherosclerosis plaque, thrombi are formed at high frequency. In addition, blood flows into the ruptured atherosclerosis plaque, whereby the plaque itself may become large. Furthermore, fatty contents flow into blood from the ruptured atherosclerosis plaque and occlude blood vessel at another site in some cases. As mentioned above, progress of atherosclerosis causes thrombosis or organ ischemia, and leads to cerebral infarction, myocardial infarction, renal failure and the like.

According to certain statistics, the causes of death in Japan are malignant neoplasms such as cancers, cardiac diseases, and cerebral vascular diseases from the top. Regardless of the statistics, cardiac diseases and cerebral vascular diseases may be caused by arteriosclerosis, and the ratio of atherosclerosis is the highest among arteriosclerosis. Consequently, it is also considered that atherosclerosis is actually the top cause of death; so it is very important to ameliorate atherosclerosis.

Antilipemic agents such as statin and antihypertensive agents such as angiotensin receptor blocking agent are generally used for atherosclerosis. However, various factors are complexly involved in generation and progress of atherosclerosis, and thus the anti-atherosclerosis agent having a mechanism of action differing from those of the above agents has been demanded.

It is considered that atherosclerosis begins by activation of monocytes in blood and invasion of the activated monocytes under vascular endothelial cells. The monocytes invaded into the intimal layer of arterial blood vessel are changed to macrophages, and are further changed to foam cells by intaking fatty materials such as cholesterol. The foam cells and transformed smooth muscle cells are migrated and accumulated, whereby atherosclerosis plaque is formed. In general, the activation of monocytes and macrophages is observed in inflammation reaction. Therefore, treatment of atherosclerosis using a factor suppressing an inflammation reaction has been investigated.

For example, it is described in Patent Document 1 to treat the diseases caused by inflammatory cytokine cascade with the antibody specifically binding to a polypeptide including A-box which constitutes a part of HMG (high mobility group protein) or to B-box which constitutes a part of HMG, to inhibit the release of pro-inflammatory cytokine. Atherosclerosis is described as one of the above mentioned diseases.

It is described in Patent Document 2 that blood disease such as atherosclerosis is treated by the antibody which binds to box of HMG.

HMGB1, i.e. High Mobility Group Box 1, is utilized as HMG in the above Patent Documents.

### PRIOR ART DOCUMENT

### PATENT DOCUMENT

Patent Document 1: JP-T-2005-512507
Patent Document 2: JP-T-2004-523579

### SUMMARY OF INVENTION

### PROBLEM TO BE SOLVED BY THE INVENTION

As mentioned above, there was an idea to treat atherosclerosis with the antibody binding to a box of HMGB1. However, the experimental result disclosed in the above-mentioned patent documents is a basic result which was obtained *in vitro.* Thus, it is not clear whether or not atherosclerosis can be actually suppressed by such an antibody.

Specifically, atherosclerosis is merely one of various diseases exemplified in Patent Document 1 as diseases related to inflammatory cytokine cascade. Further, the experimental example disclosed in Patent Document 1 mainly shows only a basic finding that an antibody binding to the B-box of HMGB1 inhibits the release of tumor necrosis factors (TNFs), and the disease for which an improved result was actually demonstrated is only sepsis.

Patent Document 2 experimentally shows only the fact that an antibody against HMGB1 inhibits transfer and morphological change of smooth muscle cell (RSMC) and the like, and does not disclose any test demonstrating that such an antibody can be actually used for the treatment of vascular diseases. Moreover, Patent Document 2 describes only *in vitro* experiments, and does not describe any results of *in vivo* experiment and clinical tests using experimental animal. In addition, although migration of smooth muscle cells is certainly a partial phenomenon of atherosclerosis, activation of monocytes and atherosclerosis plaque itself cannot be necessarily suppressed even when the migration of smooth muscle cell is inhibited.

Under the above mentioned circumstance, the problem to be solved by the present invention is to provide an agent which is useful for suppressing actual atherosclerosis and has few side-effects.

### MEANS FOR SOLVING PROBLEM

The inventors of the present invention intensively carried out study to solve the above problem. As a result, the inventors found that anti-HMGB1 monoclonal antibody which binds to C-tail of HMGB1 is very useful in actually suppressing atherosclerosis, to complete the present invention.

More specifically, monoclonal antibodies binding to A-box and B-box were previously studied as monoclonal antibody against HMGB1. The reason is considered that the box site of HMGB1 is one of binding sites for RAGE (Receptor for Advanced Glycation Endproducts) which is a multiligand receptor expressed in various cells such as smooth muscle cell and nerve cell. On the other hand, the present inventors have uniquely promoted an investigation concerning a monoclonal antibody binding to C-tail of HMGB1, and found that the monoclonal antibody binding to box of HMGB1 does not inhibit at all facilitation of vascular permeability by HMGB1 whereas a monoclonal antibody against the C-tail can significantly inhibit the facilitation. Consequently, the monoclonal antibody binding to C-tail of HMGB1 may efficiently inhibit the action of HMGB1 including acceleration of the invasion of monocyte under vascular endothelium cells. Furthermore, the present inventors experimentally demonstrated by using model animals that the monoclonal antibody can comprehensively suppress atherosclerosis.

The suppressant for atherosclerosis according to the present invention is characterized in comprising an anti-HMGB1 monoclonal antibody binding to C-tail of HMGB1 as an active component.

The method for suppressing atherosclerosis according to the present invention is characterized in comprising the step of administering an anti-HMGB1 monoclonal antibody binding to C-tail of HMGB1 as an active component.

The anti-HMGB1 monoclonal antibody according to the present invention is characterized in being used for suppressing atherosclerosis and binding to C-tail of HMGB1 as an active component. Also, the present invention relates to use of an anti-HMGB1 monoclonal antibody for producing an agent for suppressing atherosclerosis.

### BRIEF DESCRIPTION OF DRAWINGS

Fig. 1 is a graph showing the relative amount of dye (Evans blue) which extravasated from microvasculature after administration of saline, HMGB1 only, HMGB1 + the anti-HMGB1 monoclonal antibody according to the present invention, or HMGB1 + a monoclonal antibody binding to B-box of HMGB1, to the dorsal skin of experimental rats.
Fig. 2 are photographs of the sections of aortic sinus of atherosclerosis model mouse, wherein HMGB1 existing on the section was immunostained. (1) is a photograph when anti-HMGB1 antibody was used as primary antibody, and (2) is a photograph when nonspecific IgG antibody was used as primary antibody.
Fig. 3 are photographs of the cross-section of the aortic sinus slice of an atherosclerosis model mouse stained by Oil Red O. (1) is a photograph of the cross-section of a mouse to which Keyhole Limpet Hemocyanin antibody was administered as a control, and (2) is a photograph of the cross-section of a mouse to which the anti-HMGB1 antibody according to the present invention was administered. The definition is applied to Figs. 4 to 10.
Fig. 4 are photographs of the sections of aortic sinus of atherosclerosis model mice, wherein CD68 existing on the section was immunostained.
Fig. 5 are photographs of the sections of aortic sinus of atherosclerosis model mice, wherein VCAM-1 existing on the section was immunostained.
Fig. 6 are photographs of the sections of aortic sinus of atherosclerosis model mice, wherein MCP-1 existing on the section was immunostained.
Fig. 7 are photographs of the sections of aortic sinus of atherosclerosis model mice, wherein CD11c existing on the section was immunostained.
Fig. 8 are photographs of the sections of aortic sinus of atherosclerosis model mice, wherein CD83 existing on the section was immunostained.
Fig. 9 are photographs of the sections of aortic sinus of atherosclerosis model mice, wherein CD4 existing on the section was immunostained.
Fig. 10 are photographs of the sections of aortic sinus of atherosclerosis model mice, wherein PCNA existing on the section was immunostained.
Fig. 11 are graphs showing the area and ratio of stained sites, or the number of stained cells, in a case where the cross-section of the aortic sinus slice of an atherosclerosis model mouse to which IgG antibody as a control or the anti-HMGB1 antibody according to the present invention was administered was stained by Oil Red O, or in a case where antigen on the cross-section was immunostained. (1) is a case where staining was conducted using Oil Red O, and (2) to (8) are the results of cases where CD68, VCAM-1, MCP-1, CD11c, CD83, CD4 and PCNA were immunostained respectively.

### MODE FOR CARRYING OUT THE INVENTION

The suppressant for atherosclerosis according to the present invention contains an anti-HMGB1 monoclonal antibody which binds to C-tail of HMGB1 as an active component.

After the antibody of the present invention is administered, the antibody suppresses the activation of monocyte in blood and remarkably suppresses the invasion of monocyte into intimal layer. Further, the antibody suppresses macrophage foam cell formation in vascular inner wall and the expression of factor accelerating adhesion of monocyte to vascular endothelial cell. By the effects, the antibody of the present invention actually reduces the lesion of atherosclerosis. Further, it can be thought that the antibody does not effect to other compounds and therefore side-effect is none or very little according to the characteristics of monoclonal antibody.

The anti-HMGB1 monoclonal antibody of the present invention may be prepared according to conventional methods. For example, a mouse, a rat or the like is immunized using commercially available HMGB1, and the antibody-producing cell or spleen cell is fused with myeloma cell to obtain hybridoma. The hybridoma is cloned, and the clone producing an antibody which specifically reacts with C-tail of HMGB1 is screened. The clone is cultured, and secreted monoclonal antibody may be purified.

The kind of the anti-HMGB1 monoclonal antibody used in the present invention is not specifically limited. For example, a human-type antibody and a complete human antibody may be used. The antibody which is derived from an object animal to be administered is preferably used.

A dosage form of the atherosclerosis suppressant of the present invention is not specified. However, liquid preparations such as a solution and an emulsion preparation are preferable for the administration as injection, taking into consideration the fact that the anti-HMGB1 monoclonal antibody as an active ingredient is a peptide.

A solution isotonic to plasma, such as a pH-adjusted physiological saline and an aqueous solution of glucose can be used as a solvent for a liquid preparation. When the antibody is freeze-dried together with a salt or the like, pure water, distilled water, sterilized water and the like can also be used. The concentration may be that of a common antibody preparation; and may generally be about 0.1 mg/mL or more and 1 mg/mL or less, and about 0.02 mg/mL or more and 0.2 mg/mL or less for drip infusion. However, the osmotic pressure of the injection needs to be similar to that of plasma.

In the present invention, "suppression" implies both concepts of the inhibition of atherosclerosis, i.e. "prevention", and the relief or the inhibiting development of occurred atherosclerosis, i.e. "treatment". Consequently, the suppressant for atherosclerosis of the present invention may be administered for the preventive purpose between the increase of the factor developing atherosclerosis and the actual occurrence of atherosclerosis, or for the treatment purpose after the occurrence of atherosclerosis.

The frequency and the dose for administration of the suppressant for atherosclerosis of the present invention may be appropriately adjusted according to severity, age and sex of a patient. As shown in the Examples described later, a remarkable effect in suppression of atherosclerosis was obtained after administration of the anti-HMGB1 monoclonal antibody two times per week at the dose of 400 *µ*g per one time in model mice weighing about 25 g. Based on the result, the dose of the anti-HMGB1 monoclonal antibody for humans may be 0.1 mg/kg or more and 2 mg/kg or less, more preferably 0.2 mg/kg or more and 2 mg/kg or less, per one time; and administration two times per week to three times per day is acceptable.

The administration method is not limited and may be appropriately adjusted. It is preferable to administrate the anti-HMGB1 monoclonal antibody of the present invention into blood by injection, more preferably by intravenous injection, since the antibody has to be effected to monocyte in blood or atheroma sclerotic plaque.

### EXAMPLES

Hereinafter, the present invention is described in more detail with reference to the Examples. The present invention should not be naturally limited by the following Examples, and can be implemented after appropriate modification within the range compatible with the spirit of the description above and below. Such a modification is embraced by the technical scope of the present invention.

### Example 1: Preparation of the anti-HMGB1 monoclonal antibody binding to C-tail of HMGB1

### (a) Immunization of a rat

A commercially available 1 mg/mL mixture of bovine thymusderived HMGB1 and HMGB2 (manufactured by Wako Pure Chemical Industries Ltd., code No. 080-070741) was taken into a 2 mL glass syringe. An equal volume of Freund's complete adjuvant was separately taken into another 2 mL glass syringe. The mixture and the adjuvant were gradually mixed via a connecting tube, to give an emulsion. Into the footpads of the hind limbs of a sevoflurane-anesthetized rat, 0.1 mL each, 0.2 mL in total, of the emulsion was injected. Blood was sampled from the cervical vein two weeks later, and the increase of the antibody titer was confirmed. Then, the swollen iliac lymph nodes were aseptically isolated 5 weeks after the injection. About 6 × 10⁷ cells could be recovered from the two lymph nodes thus obtained.

### (b) Cell fusion and cloning

The above iliac lymph node cell and a mouse myeloma SP2/O-Ag14(SP2) cell were fused using polyethylene glycol. The resultant fused cells were distributed onto a 96-well microplate. The first ELISA screening was carried out one week later, and the cells in the positive wells were subjected to the secondary screening by Western blot analysis. The positive cells were transferred to a 24-well microplate, cultured until the cells became almost confluent (about 2 × 10⁵), and frozen for preservation in liquid nitrogen using 0.5 mL of a frozen culture medium which was a GIT medium added with 10% of bovine fetal serum and 10% of dimethylsulfoxide. The frozen cells were thawed and were cloned in a 96-well microplate.

### (c) Purification of antibody

The positive cells were cultured in large scale for 2 weeks in a rotating culture apparatus manufactured by Vivascience Co., to give an antibody fluid of a concentration of 2 to 3 mg/mL. The antibody fluid was mixed with an affinity gel: MEP-HyperCel manufactured by Invitrogen Co., at a neutral pH, so that the anti-HMGB1 antibody might be specifically bound to the gel. The antibody specifically bound to the gel was eluted with a glycine-hydrochloric acid buffer (pH 4). The eluate was concentrated in an ultrafiltration device, an antibody obtained through a Sepharose CL6B gel filtration column (2 cm in diameter × 97 cm in length) was isolated and purified.

The one antibody among the obtained monoclonal antibodies specifically recognizes an amino acid sequence in C-tail of HMGB1, 208EEEDDDDE215, in which E stands for glutamic acid and D stands for aspartic acid, as an epitope. Though HMGB2 is a protein similar to HMGB1, HMGB2 lacks the sequence: DDDDE after 211; therefore, the monoclonal antibody of the present invention does not bind to HMGB2, and can specifically recognize and bind to only HMGB1.

### Comparative Example 1: Preparation of the anti-HMGB1 monoclonal antibody binding to B-box of HMGB1

The anti-HMGB1 monoclonal antibody which recognizes B-box of HMGB1, of which sequence is LKEKYEKDIA, as an epitope was isolated and purified in a similar method of the above Example 1.

### Test Example 1: Determination of the binding site of the antibodies

The binding site of the monoclonal antibodies prepared in the above Example 1 and Comparative Example 1 was determinated once again.

Specifically, 41 peptides having 15 amino acid sequences such as 1 to 15, 6 to 20, 11 to 25 --- (as the case may be) from the N-terminus among the amino acid sequence of HMGB1 were synthesized. The 1 mg/mL solutions of the peptides were added dropwise onto Ultra Bind Membrane, manufactured by PALL Life Science, by 0.5 µL each to eliminate overlap with each other. Further, 1 mg/mL solution of purified HMG-1,2 derived from bovine thymus was also added dropwise by 0.5 µL as a positive control. After the dropwise addition, the membrane was air-dried under room temperature for 1 hour. After air drying, the membrane was blocked with a 20% skim milk/PBS solution for 1 hour. The membrane was then washed 2 times with PBS for 5 minutes, and air-dried under room temperature for 1 hour. The membrane was soaked into PBS, and each antibody solution (1 mL) diluted 500-fold with a 1% BSA/PBS solution was put into a nylon membrane and packed and was allowed to react at 4°C overnight. The membrane was then washed three times with a 0.1% Tween 20/PBS solution for 5 minutes. An anti-RAT antibody labeled with peroxidase was diluted 1000-fold with a 1% BSA/PBS solution, and the solution (1 mL) was added thereto and was allowed to react at room temperature overnight. The membrane was then washed 3 times with a 0.1% Tween 20/PBS solution for 5 minutes, and was color-developed by a chemiluminescence method.

It could be confirmed that the antibody obtained in the above Example 1 specifically binds to C-tail of HMGB1, of which sequence is EEEDDDDE, and the antibody obtained in the above Comparative Example 1 specifically binds to B-box of HMGB1, of which sequence is LKEKYEKDIA, from the amino acid sequence of which color appeared.

### Test Example 2: Vascular Permeability Test

It is said that atherosclerosis is developed when monocyte in the blood passes gaps between endothelial cells of arterial vessel and intrudes under the basal membrane of the endothelial cells, and it is thought that HMGB1 is considered to be involved in activation of monocyte and leakage of serum protein. Therefore, inhibition test of vascular permeability was carried out with HMGB1 and examined activation of each antibody.

Eight male Wistar rats weighing about 300 to 400 g were anesthetized with a gas mixture of 2% halothane, 49% oxygen and 49% laughing gas under spontaneous breathing. Subsequently, the rats were put placed on the stomach, the back fur was shaved, and ten parts with 1 cm intervals on both sides of the spine were marked. Separately, human HMGB1 recombinant was prepared by using an insect cell Sf9. Only saline (0.1 mL), only 2.5 µg/mL solution of HMGB1 (0.1 mL), 2.5 µg/mL solution of HMGB1 (0.1 mL) and 25 µg/mL solution of the anti-HMGB1 monoclonal antibody binding to C-tail (Example 1) (0.1 mL), or 2.5 µg/mL solution of HMGB1 (0.1 mL) and 25 µg/mL solution of the anti-HMGB1 monoclonal antibody binding to B-box (comparative example 1) (0.1 mL) was administered by subcutaneous injection into the every two marked spots on the rats respectively.

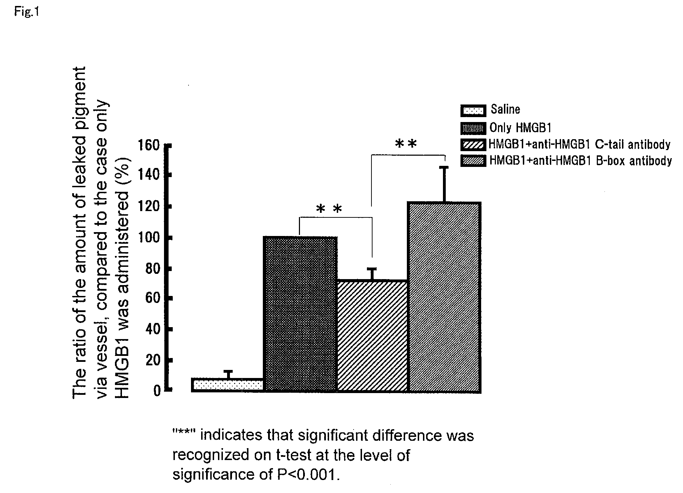

One hour after the subcutaneous injection, 2% Evans blue solution in saline was administered at the rate of 2 mL per kg weight by intravenous injection. Two hours after the intravenous injection of Evans blue, pentobarbital sodium was intraperitoneally administrated to the rats at the dose of 50 mg per kg weight so that the rats were kept in deep-anesthesia state. Saline (100mL) was infused from the left ventricle, and the blood was removed from the right atrium. Back skin of the rats was peeled off and was photographed from the rear side. The picture was analyzed by a medical image analyzing software (Image J, NIH), to measure the concentration and area of dye which was leaked out of vessel from the blood and calculate the amount thereof. The extravascular leakage amount of the dye in case where HMGB1 was administrated alone is set as 100%, and the ratios of leakage relative to 100% are shown in Fig 1.

As shown in Fig. 1, in case where only HMGB1 was administrated, the amount of leaked dye via vascular endothelial cells from the blood was naturally increased, compared to the case only saline was administrated. However, even in case of administrating the anti-HMGB1 monoclonal antibody binding to B-box in addition to HMGB1, the extravascular leakage amount of the dye tends to rather increased, although there was no significant difference.

On the other hand, in case of administrating the anti-HMGB1 monoclonal antibody binding to C-tail in addition to HMGB1, extravascular leakage of the dye was significantly suppressed at the level of significance of p<0.01, compared to not only the case of administrating HMGB1 alone but also the case of administrating the anti-HMGB1 monoclonal antibody binding to B-box.

Consequently, it was experimentally demonstrated that the anti-HMGB1 monoclonal antibody binding to C-tail according to the present invention remarkably reduces the leakage of plasma protein into atheroma sclerotic plaque and is able to suppress development and progression of atherosclerosis.

### Test Example 3: Immunohistochemical staining of HMGB1 in atherosclerotic plaque in aorta wall in mouse

### (1) Preparing atherosclerosis model mice

A six-week-aged male ApoE-/- mouse derived from C57BL, which is a general lab mouse, was obtained from the Precinct Animal Facility (AMREP, Melbourne, Australia) and fed high-fat diet containing 0.15% of cholesterol and 21% of fat for 8 weeks.

### (2) Immunostaining

The mice were killed with overdose of pentobarbitone (120 mg/kg) by intraperitoneal administration. The heart and proximal aorta were dissected from mouse, embedded in OCT compound (product name "Tissue-tek"), and frozen at -80°C. The sections of the aortic sinus having 6 µm-thick was prepared.

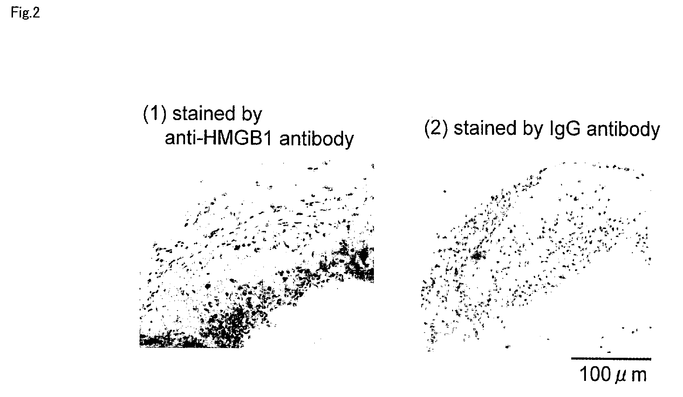

The above sections of aortic sinus were fixed with acetone at -20°C for 20 minutes. The sections were then incubated in PBS solution containing 3% of hydrogen peroxide and 10% of normal serum and biotin/avidin blocking agent manufactured by Vector Laboratories. Subsequently, the sections were incubated with a primary antibody in serum for 1 hour. Anti-HMGB1 antibody derived from rabbit (0.125 µg/mL, manufactured by BD Pharmingen, cat#556528) or nonspecific IgG antibody derived from rabbit was used as the primary antibody. Subsequently, the sections were washed and incubated with the secondary antibody for 40 minutes. Biotinylated anti-rabbit antibody (manufactured by Vector Laboratories, cat#BA-1000) was used as the secondary antibody. The sections were then incubated with streptavidin horseradish peroxidase complex (manufactured by Vector Laboratories). Antigen was visualized using 3,3-diaminobenzidine. The sections were counterstained with hematoxylin. Both photographs of the sections were shown as Fig. 2.

As shown in Fig. 2, the parts immunostained by anti-HMGB1 antibody were restricted in the intimal layer of atherosclerotic blood vessel, and the cell nuclei and cell cytoplasm existing in the parts were positively stained in the wide area. In contrast, medial cells did not express detectable HMGB1. The result indicated that HMGB1 was expressed by invaded macrophages, lymph cells, and migrated and transformed smooth muscle cell, and the like in atherosclerosis plaque; and it was found that atherosclerosis plaque was in the state that HMGB1 was provided out of the cells.

### Test Example 4: Anti-atherosclerosis Test

The effect of the anti-HMGB1 antibody of the present invention to actual atherosclerosis was examined by using atherosclerosis model mice. The experiment was approved by the Alfred Medical Research Education Precinct (AMREP) Animal Ethics Committee.

### (1) Preparing atherosclerosis model mice

Six-week-aged male ApoE-/- mice derived from C57BL, which is a general lab mouse, were obtained from the Precinct Animal Facility (AMREP, Melbourne, Australia) and fed high-fat diet containing 0.15% of cholesterol and 21% of fat for 8 weeks. The mice were also administrated either the anti-HMGB1 antibody obtained in the above Example 1 or control IgG2a against *Keyhole Limpet* hemocyanin intravenously at a dosage of 400 µg per mouse in twice a week for the duration of the dietary feeding.

### (2) Staining with Oil Red O

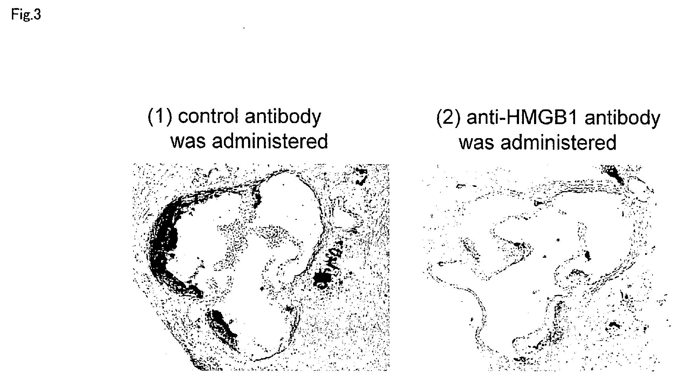

The mice were killed with overdose of pentobarbitone (120 mg/kg) by intraperitoneal administration. The heart and proximal aorta were dissected from mice, embedded in OCT compound (product name "Tissue-tek"), and frozen at -80°C. The sections of the aortic sinus having 6 µm-thick was prepared, atherosclerosis lesion part in the cross section of the aortic intima was stained with Oil Red O which can identify the fatty degeneration part. In the ascending aorta of each mouse, areas of the stained atherosclerosis lesion parts were measured at cross sections every 60 µm in the range of 180 µm from the aortic valve cusp in the ascending aorta, and the mean value thereof was calculated. Representative cross sectional pictures were shown in Fig. 3.

### (3) Immunostaining

The above sections of aortic sinus were fixed with acetone at -20°C for 20 minutes. The sections were then incubated in PBS solution containing 3% of hydrogen peroxide and 10% of normal serum and biotin/avidin blocking agent manufactured by Vector Laboratories. Subsequently, the sections were incubated with a primary antibody in serum for 1 hour. Anti-mouse CD68 antibody derived from rat (manufactured by Serotec, cat#MCA1957), anti-mouse VACM-1 antibody derived from rat (manufactured by BD Pharmingen, cat#550547), anti-rat MCP-1 antibody derived from rabbit (manufactured by Abcam, cat#ab7202), anti-mouse CD11b antibody derived from Armenian hamster (manufactured by eBioscience, cat#14-0114), anti-mouse CD83 antibody derived from rat (manufactured by eBioscience, cat#14-0831), anti-mouse CD4 antibody derived from rat (manufactured by BD Pharmingen, Cat#550274), or anti-human PCNA antibody derived from rabbit (manufactured by Abcam, cat#ab2426) was used as the primary antibody. Subsequently, the sections were washed and incubated with the secondary antibody corresponding to derivation animals for 40 minutes. As the secondary antibody, biotinylated anti-rat antibody derived from mouse (manufactured by BD Pharmingen, cat#550325), biotinylated anti-Armenian hamster antibody derived from mouse (manufactured by eBioscience, cat#13-4113-85), or biotinylated anti-rabbit antibody (manufactured by Vector Laboratories, cat#BA-1000) was used. The sections were then incubated with streptavidin horseradish peroxidase complex (manufactured by Vector Laboratories). Each antigen was visualized using 3,3-diaminobenzidine. The sections were counterstained with hematoxylin.

The parts stained with Oil Red O and the expression state of antigens were quantified by either measuring stained areas using Optimus 6.2 VideoPro-32 or counting correspondent cells.

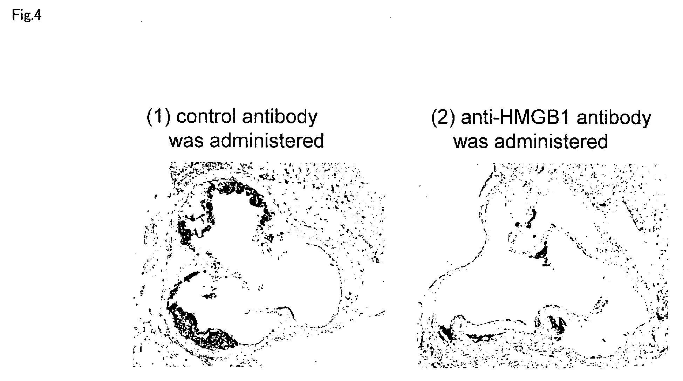

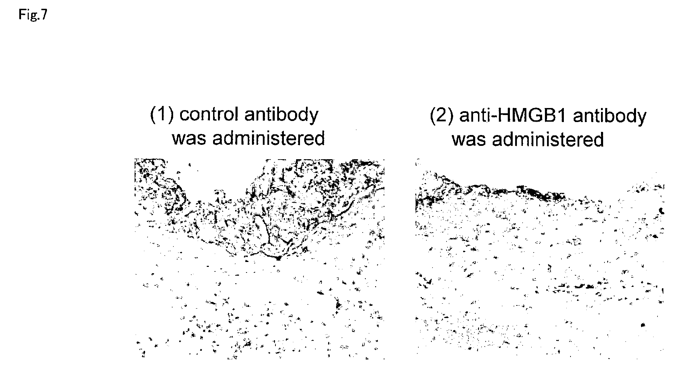

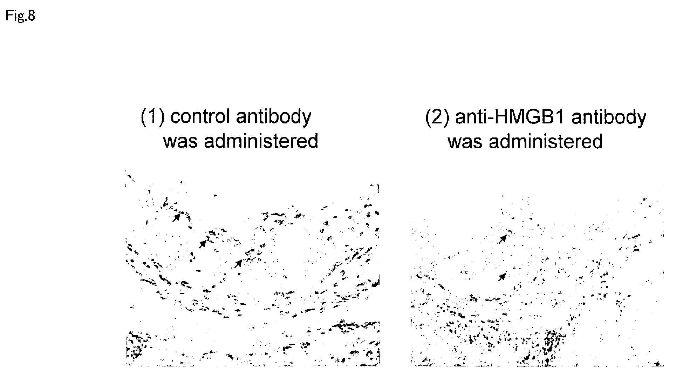

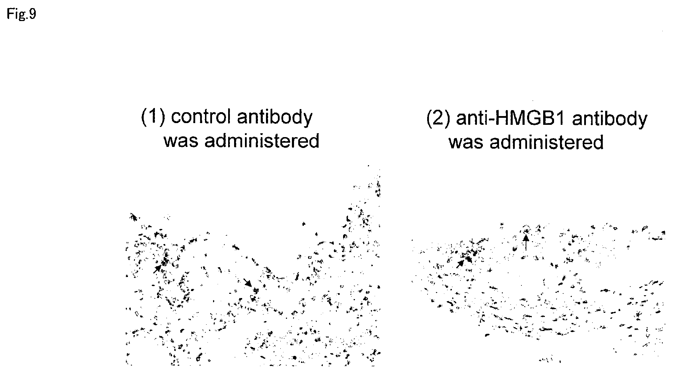

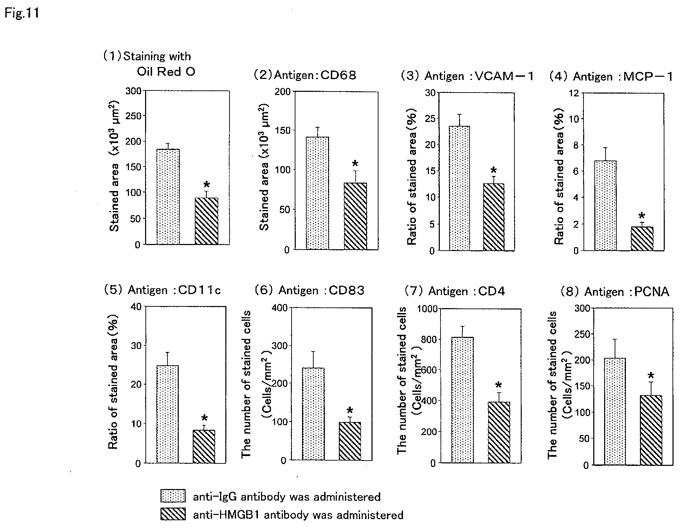

The picture of the section stained with Oil Red O is shown in Fig. 3, and the pictures of immunostained with CD68, VCAM-1, MCP-1, CD11c, CD83, CD4 and PCNA are respectively shown in Fig. 4 to 10. In Figs 3 to 10, (1) shows the result when IgG was administrated as control, (2) shows the result when the anti-HMGB1 antibody of the present invention was administrated. In addition, graphs of the results quantified the atherosclerosis lesion area per cell in each example are shown in Fig. 11. In Fig. 11, (1) shows the result when the section was stained with Oil Red O, (2) to (8) shows the result when CD68, VCAM-1, MCP-1, CD11c, CD83, CD4 and PCNA were respectively immunostained. Further, in Fig. 11, "*" indicates the case where there was a significant difference at the level of significance of p<0.05 by t-test.

The stained parts in the picture of Fig. 3 show the atherosclerosis lesion parts, since Oil Red O can stain the fatty degeneration parts. As shown in Fig. 3, the atherosclerosis lesion part is remarkably reduced when HMGB1 of the present invention is administrated in comparison with the case of administrating IgG antibody.

CD68, CD11c, CD83 and CD4 are leukocyte antigens, and are highly expressed in macrophages, immature dendritic cells, mature dendritic cells and T-lymphocytes, respectively. As shown in Figs. 4, 7 to 9 and 11, it was found that expression of the leukocyte antigens were significantly suppressed and the invasion of macrophages, dendritic cells and CD4 positive T-lymphocytes into vascular endothelial layer was significantly suppressed in a case where the anti-HMGB1 antibody according to the present invention was administered, as compared to a case where IgG antibody was administered as a control.

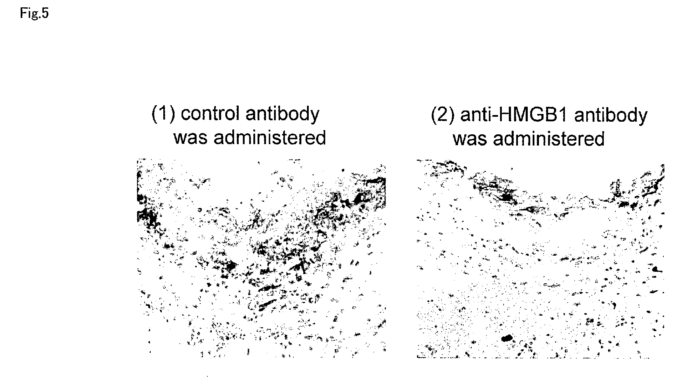

The expression level of VCAM-1 in cytoplasmic membrane is increased by activation of vascular endothelial cells. Particularly, VCAM-1 is considered to be involved in formation of arteriosclerosis. Therefore, the effect of an antibody on activation of endothelial cells can be evaluated by investigating increase and decrease of the number of VCAM-1 positive cells using immunohistochemical staining. As shown in Figs. 5 and 11, the number of VCAM-1 positive cells was decreased by half when the anti-HMGB1 antibody according to the present invention was administered, compared to the case where the control was administrated. From the result, it was revealed that activation of vascular endothelial cells which are involved in formation of arteriosclerosis can be suppressed by the antibody.

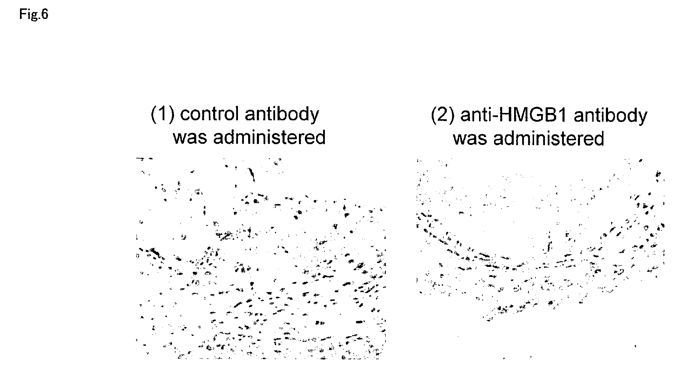

MCP-1 is a migration factor for monocytes, macrophages and dendritic cells. As shown in Fig. 6, the positive area for MCP-1 was decreased to about 30% when the anti-HMGB1 antibody according to the present invention was administered, as compared to the case where the control was administrated. From the result, it was demonstrated that the antibody can suppress migration of monocytes and the like which is involved in the formation of arteriosclerosis.

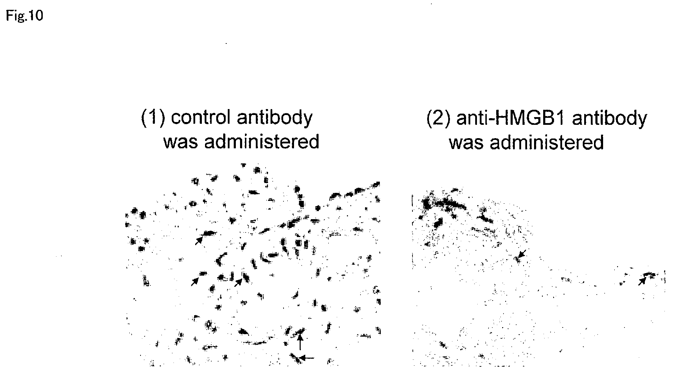

PCNA is a nuclear factor which is involved in synthesis and repair of DNA. It is considered that PCNA is highly expressed in proliferative cells in the migratory smooth muscles in the vascular wall. As shown in Figs. 10 and 11, the number of PCNA-positive cells was decreased by about 35% when the anti-HMGB1 antibody according to the present invention was administered, as compared to the case where the control was administrated. From the result, it was found that arteriosclerosis is suppressed by the antibody.

### INDUSTRIAL APPLICABILITY

The agent according to the present invention can effectively suppress atherosclerosis, which causes various organ disorders such as cerebral infarction and myocardial infarction, by inhibiting accumulation of activated monocytes on vascular intima layer and other actions. Further, probability of serious side-effects by the agent is considered to be quite low in view of the currently used antibody drugs. Therefore, the atherosclerosis suppressant according to the present invention is extremely useful as a practical drug capable of suppressing atherosclerosis by a mechanism of action different from those of drugs such as antilipemic agent and antihypertensive agent which have been put to practical use so far.

## Claims

1. An anti-HMGB1 monoclonal antibody for use in preventing atherosclerosis, wherein the antibody specifically recognizes the amino acid sequence EEEDDDDE in the C-tail of HMBG1.

2. The anti-HMGB1 monoclonal antibody for use according to claim 1, wherein the anti-HMGB1 monoclonal antibody is administered by intravenous injection.

3. The anti-HMGB1 monoclonal antibody for use according to claim 2, wherein the anti-HMGB1 monoclonal antibody is administered at a dose of not less than 0.2 mg/kg and not more than 2 mg/kg per one time.

## Patentansprüche

1. Monoklonaler anti-HMGB1 Antikörper zur Verwendung in der Vorbeugung von Atherosklerose, wobei der Antikörper spezifisch die Aminosäuresequenz EEEDDDDE im C-Terminus von HMBG1 erkennt.

2. Monoklonaler anti-HMGB1 Antikörper zur Verwendung nach Anspruch 1, wobei der monoklonale anti-HMGB1 Antikörper durch intravenöse Injektion verabreicht wird.

3. Monoklonaler anti-HMGB1 Antikörper zur Verwendung nach Anspruch 2, wobei der monoklonale anti-HMGB1 Antikörper pro einem Mal bei einer Dosis von nicht weniger als 0,2 mg/kg und nicht mehr als 2 mg/kg verabreicht wird.

## Revendications

1. Anticorps monoclonal anti-HMGB1 pour une utilisation dans la prévention de l'athérosclérose, dans lequel l'anticorps reconnaît spécifiquement la séquence d'acides aminés EEEDDDDE dans la queue en C de la HMGB1.

2. Anticorps monoclonal anti-HMGB1 pour l'utilisation selon la revendication 1, dans lequel l'anticorps monoclonal anti-HMGB1 est administré par injection intraveineuse.

3. Anticorps monoclonal anti-HMGB1 pour l'utilisation selon la revendication 2, dans lequel l'anticorps monoclonal anti-HMGB1 est administré à une dose qui n'est pas inférieure à 0,2 mg/kg et qui n'est pas supérieure à 2 mg/kg à chaque fois.
